# EUROPÄISCHE PATENTSCHRIFT

(11) **EP 3 359 632 B1**
(45) Veröffentlichungstag und Bekanntmachung des Hinweises auf die Patenterteilung: **11.12.2019**
(21) Anmeldenummer: 16778389.3
(22) Anmeldetag: 07.10.2016
(51) Int. Cl.: C11D 3/30, C11D 9/30, A61K 8/41

(54) **ZUSAMMENSETZUNGEN ENTHALTEND ZUCKERAMIN UND FETTSÄURE**
COMPOSITIONS COMPRISING SUGAR AMINE AND FATTY ACID
COMPOSITIONS CONTENANT UNE AMINE DE SUCRE ET UN ACIDE GRAS

(30) Priorität: 09.10.2015 DE 102015219651
(43) Veröffentlichungstag der Anmeldung: 15.08.2018
(73) Patentinhaber: Clariant International Ltd, 4132 Muttenz (CH)
(72) Erfinder: KLUG, Peter, 63762 Grossostheim (DE); COHRS, Carsten, 60316 Frankfurt am Main (DE); BACK, Ute, 63825 Blankenbach (DE); MUTCH, Kevin, 60385 Frankfurt (DE)
(74) Vertreter: Paczkowski, Marcus
(86) Internationale Anmeldenummer: PCT/EP2016/074085
(87) Internationale Veröffentlichungsnummer: WO 2017/060481

(56) Entgegenhaltungen:
- EP-A1- 0 039 860
- EP-A1- 1 422 288
- DE-A1- 19 846 429
- US-A- 4 481 186
- US-A1- 2011 263 471
- DATABASE WPI Week 201016 Thomson Scientific, London, GB; AN 2010-B81401 XP002765314, & JP 2010 037252 A (MEDREX KK) 18. Februar 2010 (2010-02-18)

## Beschreibung

Die Erfindung betrifft Zusammensetzungen enthaltend ein oder mehrere Zuckeramine und/oder protonierte Zuckeramine mit Fettsäureanionen als Gegenionen und ein oder mehrere Fettsäuren und/oder Fettsäuresalze, die Verwendung derartiger Zusammensetzungen zur Herstellung von Flüssigwaschmitteln oder von Duschbädern, Flüssigseifen oder Gesichtsreinigern sowie die Verwendung der Zuckeramine als Neutralisationsmittel für Fettsäuren. Die Erfindung betrifft weiterhin Salze aus Zuckeraminen und Fettsäuren.

Die Verwendung von Zuckeraminen in Waschmitteln und Kosmetika ist bereits aus dem Stand der Technik bekannt.

In DE 42 38 211 C1 werden Detergensgemische enthaltend spezielle kationische Zuckertenside, beispielsweise abgeleitet von Glucaminen, beschrieben und deren Verwendung zur Herstellung von z.B. Waschmitteln und Produkten zur Haar- und Körperpflege. Die Detergensgemische zeichnen sich u.a. durch eine hohe Kaltwasserlöslichkeit bzw. Kaltwasserdispergierbarkeit aus und lassen sich problemlos zu hochkonzentrierten, niedrigviskosen Lösungen bzw. Dispersionen verarbeiten.

In DE 198 08 824 C1 werden Haarbehandlungsmittel mit einem Gehalt an mindestens einem Polymer mit sauren Gruppen, wie z.B. Carbonsäuregruppen, und mindestens einem N-unsubstituierten oder N-substituierten Polyhydroxyamin mit mindestens vier Hydroxylgruppen, wie z.B. Glucamin, offenbart und darüber hinaus die Verwendung von mindestens einem N-unsubstituierten oder N-substituierten Polyhydroxyamin mit mindestens vier Hydroxylgruppen in Haarbehandlungsmitteln zur Neutralisation von Polymeren mit sauren Gruppen.

In EP 1 676 831 A1 werden am Stickstoff disubstituierte Polyhydroxyalkylamine, wie z.B. N,N-Dialkylglucamine, beschrieben, wobei beide Substituenten jeweils 2 bis 30 Kohlenstoffatome enthalten. Des Weiteren wird auch eine Vielzahl von Anwendungsmöglichkeiten für die am Stickstoff disubstituierten

Polyhydroxyalkylamine offenbart wie z.B. die Verwendung in wässrigen Flüssigwaschmitteln oder in Shampoos, flüssigen Körperreinigungsformulierungen, Haarkonditionierformulierungen und wässrigen Sonnenschutzformulierungen.

In EP 1 529 832 A1 werden Metallbearbeitungsflüssigkeiten enthaltend Aminopolyole wie beispielsweise D-Glucamin, Methylglucamin, Ethylglucamin oder Hydroxyethylglucamin offenbart. Darüber hinaus wird beispielsweise beschrieben, dass Aminopolyole und vorzugsweise Aminozucker z.B. als Neutralisations- und Reaktionskomponente in Anstrichmitteln, Kosmetika, physiologischen Lösungen und Trägerstoffen für Arzneimittel verwendet werden können.

In EP 614 881 wird die Herstellung von tertiären Dialkylpolyhydroxyaminen, wie z.B. Dimethylglucamin, beschrieben.

In JP 2010-037252 A werden wässrige Wirkstoffdispersionen für intravenöse Injektionen beschrieben, in denen der Wirkstoff in einem wässrigen Medium gelöst wird, das Methylglucamin-Salze enthält. Die Offenlegungsschriften US 2011/0263471 A1 und EP 1 422 288 A1 offenbaren Handwaschmittel bzw. flüssige Reinigungsmittel, die Fettsäuren und Methylglucamin enthalten können. In US 4,481,186 werden kosmetische Emulsionen beschrieben, die Glucamine als auch Fettsäuren enthalten. Die DE 198 46 429 A1 hat Stückseifen zum Gegenstand, die neben Fettsäuresalzen auch Glucammonium-Salz enthalten können. In EP 0 039 860 A1 werden aminierte Saccharide als Tenside für kosmetische Zusammensetzungen offenbart.

Fettsäuren bzw. deren Salze (wobei die Salze der Fettsäuren auch als "Fettsäureseifen" bezeichnet werden) sind sehr preiswerte Inhaltsstoffe für Wasch- und Reinigungsmittel. Jedoch sind typische Seifenlösungen erst bei relativ hohem pH-Wert homogen und oft relativ viskos und weisen speziell bei tendenziell niedrigeren pH-Werten mangelnde Löslichkeit auf.

Es bestand daher die Aufgabe, neue Zusammensetzungen zur Verfügung zu stellen, die über einen breiten pH-Bereich niedrigviskose, homogene Lösungen von Fettsäuren oder Fettsäureseifen in wässrigen Zusammensetzungen ergeben und somit die Einarbeitung von längerkettigen Fettsäuren und/oder Fettsäureseifen in Wasch- und Reinigungsmittel erleichtern.

Es wurde nun überraschenderweise gefunden, dass diese Aufgabe gelöst wird durch Zusammensetzungen enthaltend
a) eine oder mehrere Substanzen ausgewählt aus der Gruppe bestehend aus den Zuckeraminen Dimethylglucamin und Hydroxyethyl-methyl-Glucamin, den protonierten Zuckeraminen Dimethylglucammonium mit dem Gegenion R-COO⁻ und Hydroxyethyl-methyl Glucammonium mit dem Gegenion R-COO⁻ und Mischungen davon
   wobei
   - R: die Bedeutung von R aus den Substanzen der Komponente b) unten besitzt
   und
b) eine oder mehrere Substanzen ausgewählt aus der Gruppe bestehend aus Fettsäuren der Formel R-COOH, Fettsäuresalzen der Formel R-COO⁻M⁺ und Mischungen davon, worin
   R einen linearen oder verzweigten gesättigten Alkylrest mit 11 bis 21 Kohlenstoffatomen oder einen linearen oder verzweigten einfach oder mehrfach ungesättigten Alkenylrest mit 11 bis 21 Kohlenstoffatomen bedeutet und
   M⁺ ein Gegenion ist, vorzugsweise ausgewählt aus der Gruppe bestehend aus NH₄⁺, organischen Ammoniumionen [HNR⁵R⁶R⁷]⁺, wobei R⁵, R⁶ und R⁷ unabhängig voneinander Wasserstoff, eine lineare oder verzweigte gesättigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine C₆-C₂₂-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste R⁵, R⁶ und R⁷ nicht Wasserstoff ist, Li⁺, Na⁺, K⁺, ½ Ca⁺⁺, ½ Mg⁺⁺, ½ Zn⁺⁺ oder ⅓ Al⁺⁺⁺ und Mischungen aus diesen Ionen.

Gegenstand der Erfindung sind daher die entsprechenden Zusammensetzungen gemäß Anspruch 1 enthaltend die Komponenten a) und b).

Wie bereits erwähnt weisen die erfindungsgemäßen Zusammensetzungen beispielsweise den Vorteil auf, dass sie über einen breiten pH-Bereich niedrigviskose, homogene Lösungen von Fettsäuren oder Fettsäuresalzen in wässrigen Zusammensetzungen ergeben.

In den erfindungsgemäßen Zusammensetzungen besitzt der Rest R in den protonierten Zuckeraminen die Bedeutung von R aus den Substanzen der Komponente b) der erfindungsgemäßen Zusammensetzungen. Dies bedeutet, dass der Rest R in den protonierten Zuckeraminen auch dann die Bedeutung von R aus den Substanzen der Komponente b) der erfindungsgemäßen Zusammensetzungen annimmt, wenn R in den Substanzen der Komponente b) der erfindungsgemäßen Zusammensetzungen bevorzugte, besonders bevorzugte, insbesondere bevorzugte Bedeutungen etc. annimmt.

Die eine oder die mehreren Substanzen der Komponente a) der erfindungsgemäßen Zusammensetzungen sind ausgewählt aus der Gruppe bestehend aus Dimethylglucamin, Hydroxyethyl-methyl Glucamin, Dimethylglucammonium mit dem Gegenion R-COO⁻, Hydroxyethyl-methyl Glucammonium mit dem Gegenion R-COO⁻ und Mischungen davon.

Bei Dimethylglucammonium mit dem Gegenion R-COO⁻ handelt es sich um Verbindungen der Formel (Ia) 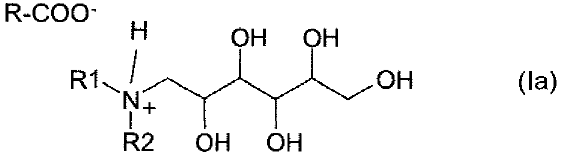 worin R1 und R2 CH₃ bedeuten.

Bei Hydroxyethyl-methyl Glucammonium mit dem Gegenion R-COO⁻ handelt es sich um Verbindungen der Formel (Ia), worin einer der Reste R1 oder R2 CH₃ bedeutet und der andere dieser Reste -CH₂CH₂-OH bedeutet.

Besonders bevorzugt ist die eine oder sind die mehreren Substanzen der Komponente a) ausgewählt aus der Gruppe bestehend aus Dimethylglucamin, Dimethylglucammonium mit dem Gegenion R-COO⁻ und Mischungen davon.

Vorzugsweise ist das Gegenion M⁺ der Fettsäuresalze der Formel R-COO⁻M⁺ aus Komponente b) der erfindungsgemäßen Zusammensetzungen ausgewählt aus der Gruppe bestehend aus NH₄⁺, Monoethanolammonium, Diethanolammonium, Triethanolammonium, Methylpropanolammonium, Na⁺, K⁺ und Mischungen aus diesen Ionen und besonders bevorzugt ausgewählt aus der Gruppe bestehend aus Na⁺, K⁺ und Mischungen aus diesen Ionen.

Vorzugsweise ist die eine oder sind die mehreren Substanzen der Komponente b) der erfindungsgemäßen Zusammensetzungen ausgewählt aus der Gruppe bestehend aus den Fettsäuren (R-COOH) Laurinsäure, Myristinsäure, Palmitinsäure, Stearinsäure, Ölsäure, Linolsäure, C₁₆/C₁₈ Fettsäuren, Behensäure und Erucasäure, den Salzen der genannten Fettsäuren mit M⁺ als Gegenion (R-COO⁻M⁺) und Mischungen davon. Die soeben beschriebene bevorzugte Ausführungsform der Erfindung wird im Folgenden als "Ausführungsform A" bezeichnet.

Besonders bevorzugt ist die eine oder sind die mehreren Substanzen der Komponente b) der erfindungsgemäßen Zusammensetzungen ausgewählt aus der Gruppe bestehend aus C₁₆/C₁₈ Fettsäuren, den Salzen von C₁₆/C₁₈ Fettsäuren mit M⁺ als Gegenion und Mischungen davon. Die soeben beschriebene besonders bevorzugte Ausführungsform der Erfindung wird im Folgenden als "Ausführungsform B" bezeichnet.

Insbesondere bevorzugt ist die eine oder sind die mehreren Substanzen der Komponente b) der erfindungsgemäßen Zusammensetzungen ausgewählt aus der Gruppe bestehend aus Ölsäure, den Salzen von Ölsäure mit M⁺ als Gegenion und Mischungen davon. Die soeben beschriebene insbesondere bevorzugte Ausführungsform der Erfindung wird im Folgenden als "Ausführungsform C" bezeichnet.

In einer außerordentlich bevorzugten Ausführungsform der Erfindung ist die eine oder sind die mehreren Substanzen der Komponente a) der erfindungsgemäßen Zusammensetzungen ausgewählt aus der Gruppe bestehend aus Dimethylglucamin, Dimethylglucammonium mit dem Gegenion R-COO⁻, worin R-COO⁻ dem Ölsäureanion (bzw. deprotonierter Ölsäure) entspricht, und Mischungen davon und ist die eine oder sind die mehreren Substanzen der Komponente b) der erfindungsgemäßen Zusammensetzungen ausgewählt aus der Gruppe bestehend aus Ölsäure, den Salzen von Ölsäure mit M⁺ als Gegenion und Mischungen davon. Die soeben beschriebene außerordentlich bevorzugte Ausführungsform der Erfindung wird im Folgenden als "Ausführungsform D" bezeichnet.

In den Ausführungsformen A, B, C und D ist M⁺ ein Gegenion, vorzugsweise ist es ausgewählt aus der Gruppe bestehend aus NH₄⁺, organischen Ammoniumionen [HNR⁵R⁶R⁷]⁺, wobei R⁵, R⁶ und R⁷ unabhängig voneinander Wasserstoff, eine lineare oder verzweigte gesättigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine C₆-C₂₂-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste R⁵, R⁶ und R⁷ nicht Wasserstoff ist, Li⁺, Na⁺, K⁺, ½ Ca⁺⁺, ½ Mg⁺⁺, ½ Zn⁺⁺ oder ⅓ Al⁺⁺⁺ und Mischungen aus diesen Ionen, besonders bevorzugt ist es ausgewählt aus der Gruppe bestehend aus NH₄⁺, Monoethanolammonium, Diethanolammonium, Triethanolammonium, Methylpropanolammonium, Na⁺, K⁺ und Mischungen aus diesen Ionen und insbesondere bevorzugt ist es ausgewählt aus der Gruppe bestehend aus Na⁺, K⁺ und Mischungen aus diesen Ionen.

Unter den erfindungsgemäßen Zusammensetzungen sind diejenigen bevorzugt, in denen Komponente a) ein oder mehrere protonierte Zuckeramine der Formel (la) enthält. In diesen bevorzugten erfindungsgemäßen Zusammensetzungen sind die Zuckeramine Dimethylglucamin und Hydroxyethyl-methyl-Glucamin aus Komponente a) lediglich optional enthalten.

Unter den erfindungsgemäßen Zusammensetzungen sind in einer bevorzugten Ausführungsform der Erfindung diejenigen bevorzugt, in denen Komponente b) eine oder mehrere Fettsäuren der Formel R-COOH enthält. In diesen bevorzugten erfindungsgemäßen Zusammensetzungen sind die Fettsäuresalze der Formel R-COO⁻M⁺ aus Komponente b) lediglich optional enthalten. Unter diesen bevorzugten erfindungsgemäßen Zusammensetzungen sind in einer besonders bevorzugten Ausführungsform der Erfindung wiederum diejenigen bevorzugt, in denen Komponente b) keine Fettsäuresalze der Formel R-COO⁻M⁺ enthält.

Vorzugsweise enthalten die erfindungsgemäßen Zusammensetzungen Wasser.

Die Substanzen der Formel (la) aus Komponente a) und die Substanzen der Komponente b) der erfindungsgemäßen Zusammensetzungen stellen Tenside dar. Hierbei stellen nicht nur die Substanzen der Formel (la) und die Fettsäuresalze der Formel R-COO⁻M⁺ aus Komponente b) der erfindungsgemäßen Zusammensetzungen anionische Tenside dar, sondern im Rahmen der vorliegenden Erfindung werden auch die Fettsäuren R-COOH der Komponente b) der erfindungsgemäßen Zusammensetzungen als anionische Tenside betrachtet, Dimethylglucamin und Hydroxyethyl-methyl-Glucamin aus Komponente a) der erfindungsgemäßen Zusammensetzungen stellen dagegen keine Tenside dar.

Neben den Substanzen der Formel (la) aus Komponente a) und den Substanzen der Komponente b) enthalten die erfindungsgemäßen Zusammensetzungen vorzugsweise ein oder mehrere weitere Tenside.

Als derartige weitere Tenside werden im Rahmen der vorliegenden Erfindung Substanzen bezeichnet, die die Oberflächenspannung einer Flüssigkeit oder die Grenzflächenspannung zwischen zwei Phasen herabsetzen und die Bildung von Dispersionen oder Emulsionen ermöglichen oder unterstützen. Dies bedeutet insbesondere, dass der Begriff "Tenside" im Rahmen der vorliegenden Erfindung auch Substanzen umfasst, die üblicherweise als Emulgatoren bezeichnet werden.

Die weiteren Tenside können vorteilhafterweise ausgewählt werden aus der Gruppe bestehend aus nichtionischen Tensiden, anionischen Tensiden, kationischen Tensiden, amphotheren Tensiden und Betaintensiden.

Die Menge der in den erfindungsgemäßen Zusammensetzungen enthaltenen Tenside (inklusive der Substanzen der Komponente b) und der Substanzen der Formel (la) aus Komponente a) der erfindungsgemäßen Zusammensetzungen) beträgt, bezogen auf das Gesamtgewicht der erfindungsgemäßen Zusammensetzungen, vorzugsweise von 0,01 bis 60,0 Gew.-%, besonders bevorzugt von 1,0 bis 40,0 Gew.-%, insbesondere bevorzugt von 2,0 bis 30,0 Gew.-% und außerordentlich bevorzugt von 3,0 bis 15,0 Gew.-%.

Als weitere anionische Tenside bevorzugt sind (C₁₀-C₂₂)-Alkylethercarboxylate, Fettalkoholsulfate wie z. B. Laurylsulfat, Fettalkoholethersulfate wie z. B. Laurylethersulfat (oder Laurethsulfat), Alkylamidsulfate, Alkylamidsulfonate, Fettsäurealkylamidpolyglykolethersulfate, Alkansulfonate, Hydroxyalkansulfonate, Olefinsulfonate, Acylester von Isethionaten, α-Sulfofettsäureester (Methylestersulfonate), Alkylbenzolsulfonate, bevorzugt lineare Alkylbenzolsulfonate, Alkylphenolglykolethersulfonate, Sulfosuccinate, Sulfobernsteinsäurehalbester, Sulfobernsteinsäurediester, Fettalkoholphosphate, Fettalkoholetherphosphate, Eiweiß-Fettsäure-Kondensationsprodukte, Alkylmonoglyceridsulfate, Alkylmonoglyceridsulfonate, Alkylglyceridethersulfonate, Fettsäuremethyltauride, Fettsäuresarkosinate, Sulforicinoleate, Acylglutamate wie z. B. Natriumcocoylglutamat und Acylglycinate. Diese Verbindungen und deren Mischungen werden in Form ihrer wasserlöslichen oder in Wasser dispergierbaren Salze benutzt, beispielsweise in Form ihrer Natrium-Salze, Kalium-Salze, Magnesium-Salze, Ammonium-Salze, Monoethanolammonium-Salze, Diethanolammonium-Salze und/oder Triethanolammonium-Salze sowie der analogen Alkylammonium-Salze.

Die Menge der anionischen Tenside in den erfindungsgemäßen Zusammensetzungen (inklusive der Substanzen der Komponente b) und der Substanzen der Formel (la) aus Komponente a) der erfindungsgemäßen Zusammensetzungen) beträgt, bezogen auf das Gesamtgewicht der erfindungsgemäßen Zusammensetzungen, vorzugsweise von 0,1 bis 30,0 Gew.-%, besonders bevorzugt von 0,2 bis 20,0 Gew.-% und insbesondere bevorzugt von 0,5 bis 15,0 Gew.-%.

Bevorzugte kationische Tenside sind quartäre Ammonium-Salze wie
Di-(C₈-C₂₂)-Alkyl-dimethylammoniumchlorid oder -bromid;
(C₈-C₂₂)-Alkyl-dimethyl-ethylammoniumchlorid oder -bromid;
(C₈-C₂₂)-Alkyl-trimethylammoniumchlorid oder -bromid, vorzugsweise
Cetyltrimethylammoniumchlorid oder -bromid;
(Cio-C₂₄)-Alkyl-dimethylbenzyl-ammoniumchlorid oder -bromid, vorzugsweise
(C₁₂-C₁₈)-Alkyl-dimethylbenzyl-ammoniumchlorid;
(C₈-C₂₂)-Alkyl-dimethyl-hydroxyethylammoniumchlorid, -phosphat, -sulfat oder -lactat; (C₈-C₂₂)-Alkylamidopropyltrimethylammoniumchlorid oder -methosulfat;
N,N-bis(2-C₈-C₂₂-Alkanoyl-oxyethyl)-dimethylammoniumchlorid oder-methosulfat
sowie N,N-bis(2-C₈-C₂₂-Alkanoyl-oxyethyl)hydroxyethyl-methyl-ammoniumchlorid oder -methosulfat.

Die Menge der kationischen Tenside in den erfindungsgemäßen Zusammensetzungen beträgt, bezogen auf das Gesamtgewicht der erfindungsgemäßen Zusammensetzungen, vorzugsweise von 0,1 bis 10,0 Gew.-%, besonders bevorzugt von 0,5 bis 7,0 Gew.-% und insbesondere bevorzugt von 1,0 bis 5,0 Gew.-%.

Als nichtionische Tenside bevorzugt sind Fettalkoholethoxylate (Alkylpolyethylenglykole); Alkylphenolpolyethylenglykole; Fettaminethoxylate (Alkylaminopolyethylenglykole); Fettsäureethoxylate (Acylpolyethylenglykole); Polypropylenglykolethoxylate (Pluronics®); Fettsäurealkanolamide, (Fettsäureamidpolyethylenglykole); N-Acyl-N-methylglucamine; Saccharoseester; Sorbitester und Sorbitanester und deren Polyglykolether; sowie C₈-C₂₂-Alkylpolyglucoside.

Die Menge der nichtionischen Tenside in den erfindungsgemäßen Zusammensetzungen beträgt, bezogen auf das Gesamtgewicht der erfindungsgemäßen Zusammensetzungen, vorzugsweise von 1,0 bis 20,0 Gew.-%, besonders bevorzugt von 2,0 bis 10,0 Gew.-% und insbesondere bevorzugt von 3,0 bis 7,0 Gew.-%.

Weiterhin können die erfindungsgemäßen Zusammensetzungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z. B. mit einer Carboxyl-Gruppe, Sulfat-Gruppe oder Sulfonat-Gruppe. Bevorzugte Amphotenside sind Aminopropionate wie N-(C₁₂-C₁₈)-Alkyl-β-aminopropionate und N-(C₁₂-C₁₈)-Alkyl-β-iminodipropionate als Alkali-Salze, Monoalkylammonium-Salze, Dialkylammonium-Salze oder Trialkylammonium-Salze sowie Lauroamphoacetat, insbesondere als Natriumsalz.

Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die C₁₀- bis C₁₈-Alkyldimethylaminoxide und Fettsäureamidoalkyldimethylaminoxide.

Eine weitere bevorzugte Gruppe von Tensiden sind Betaintenside, auch zwitterionische Tenside genannt. Diese enthalten im selben Molekül eine kationische Gruppe, insbesondere eine Ammonium-Gruppe und eine anionische Gruppe, die eine Carboxylat-Gruppe, Sulfat-Gruppe oder Sulfonat-Gruppe sein kann. Geeignete Betaine sind vorzugsweise Alkylbetaine wie Coco-Betain oder Fettsäurealkylamidopropylbetaine (bzw. Alkylamidopropylbetaine), beispielsweise Kokosacylamidopropyldimethylbetain (bzw. Cocoamidopropylbetain).

Die Menge der amphoteren Tenside und/oder Betaintenside in den erfindungsgemäßen Zusammensetzungen beträgt, bezogen auf das Gesamtgewicht der erfindungsgemäßen Zusammensetzungen, vorzugsweise von 0,5 bis 20,0 Gew.-% und besonders bevorzugt von 1,0 bis 15,0 Gew.-%.

In einer bevorzugten Ausführungsform der Erfindung enthalten die erfindungsgemäßen Zusammensetzungen ein oder mehrere Tenside ausgewählt aus der Gruppe bestehend aus Laurylsulfat, Laurethsulfat, Methylestersulfonat, Cocoamidopropylbetain, Alkylbetainen wie Coco-Betain, Natriumcocoylglutamat und Lauroamphoacetat.

In einer weiteren bevorzugten Ausführungsform der Erfindung enthalten die erfindungsgemäßen Zusammensetzungen ein oder mehrere Tenside ausgewählt aus der Gruppe bestehend aus alkylierten Ethersulfaten mit einer linearen oder verzweigten Alkylgruppe mit 8 bis 30 Kohlenstoffatomen oder einer linearen oder verzweigten einfach oder mehrfach ungesättigten Alkenylgruppe mit 8 bis 30 Kohlenstoffatomen, Betainen und deren Derivaten sowie Mischungen davon und insbesondere bevorzugt ausgewählt aus der Gruppe bestehend aus alkylierten Ethersulfaten mit einer linearen oder verzweigten Alkylgruppe mit 12 bis 22 Kohlenstoffatomen, Betainen und deren Derivaten sowie Mischungen davon.

In einer weiteren bevorzugten Ausführungsform der Erfindung enthalten die erfindungsgemäßen Zusammensetzungen neben den Substanzen der Formel (la) aus Komponente a) und den Substanzen der Komponente b) ein oder mehrere weitere anionische Tenside. Hierunter wiederum bevorzugt ist das eine oder sind die mehreren weiteren anionischen Tenside ausgewählt aus der Gruppe bestehend aus Alkyl- oder Alkenylpolyglykolethersulfat, vorzugsweise Alkylpolyglykolethersulfat, Alkyl- oder Alkenylsulfat, vorzugsweise Alkylsulfat, Methylestersulfonat, Alkylbenzolsulfonat, Acylglutamat und Mischungen davon. Bei den soeben genannten ein oder mehreren weiteren anionischen Tensiden bedeutet "Alkyl" vorzugsweise eine lineare oder verzweigte gesättigte Alkylgruppe mit 12 bis 22 Kohlenstoffatomen, "Alkenyl" eine lineare oder verzweigte einfach oder mehrfach ungesättigte Alkenylgruppe mit 12 bis 22 Kohlenstoffatomen und "Acyl" eine lineare oder verzweigte gesättigte Acylgruppe mit 12 bis 22 Kohlenstoffatomen oder eine lineare oder verzweigte ein- oder mehrfach ungesättigte Acylgruppe mit 12 bis 22 Kohlenstoffatomen.

In einer weiteren bevorzugten Ausführungsform der Erfindung enthalten die erfindungsgemäßen Zusammensetzungen ein oder mehrere nichtionische Tenside ausgewählt aus der Gruppe bestehend aus Alkyl- oder Alkenylethoxylaten, vorzugsweise Alkylethoxylaten, Alkylpolyglucosiden, Acyl-N-Methylglucaminen, Fettsäurealkanolamiden wie beispielsweise Cocosfettsäuremonoethanolamid und Mischungen davon. Bei den soeben genannten ein oder mehreren nichtionischen Tensiden bedeutet "Alkyl" vorzugsweise eine lineare oder verzweigte gesättigte Alkylgruppe mit 12 bis 22 Kohlenstoffatomen, "Alkenyl" eine lineare oder verzweigte einfach oder mehrfach ungesättigte Alkenylgruppe mit 12 bis 22 Kohlenstoffatomen und "Acyl" eine lineare oder verzweigte gesättigte Acylgruppe mit 12 bis 22 Kohlenstoffatomen oder eine lineare oder verzweigte einfach oder mehrfach ungesättigte Acylgruppe mit 12 bis 22 Kohlenstoffatomen. Die Fettsäuregruppen besitzen vorzugsweise Acylgruppen wie soeben für "Acyl" definiert.

Vorzugsweise enthalten die erfindungsgemäßen Zusammensetzungen, bezogen auf das Gesamtgewicht der erfindungsgemäßen Zusammensetzungen, 0,5 bis 30 Gew.-%, besonders bevorzugt 0,5 bis 20 Gew.-%, insbesondere bevorzugt 1 bis 15 Gew.-% und außerordentlich bevorzugt 1 bis 10 Gew.-% an der einen oder den mehreren Substanzen der Komponente a).

Vorzugsweise enthalten die erfindungsgemäßen Zusammensetzungen, bezogen auf das Gesamtgewicht der erfindungsgemäßen Zusammensetzungen, 0,5 bis 30 Gew.-% und besonders bevorzugt 1 bis 10 Gew.-% an der einen oder den mehreren Substanzen der Komponente b).

Vorzugsweise ist das molare Verhältnis Komponente (i): Komponente (ii) in den erfindungsgemäßen Zusammensetzungen von 5 : 1 bis 1 : 5 und besonders bevorzugt von 3 : 1 bis 1 : 3, wobei hierbei Komponente (i) aus der einen oder den mehreren Substanzen der Komponente b) der erfindungsgemäßen Zusammensetzungen und dem einen oder den mehreren Anionen RCOO⁻ der einen oder der mehreren protonierten Zuckeramine aus Komponente a) der erfindungsgemäßen Zusammensetzungen besteht und Komponente (ii) aus dem einen oder den mehreren Zuckeraminen aus Komponente a) der erfindungsgemäßen Zusammensetzungen und dem einen oder den mehreren Kationen der protonierten Zuckeramine aus Komponente a) der erfindungsgemäßen Zusammensetzungen besteht.

In einer besonders bevorzugten Ausführungsform der Erfindung ist das molare Verhältnis Komponente (i): Komponente (ii) in den erfindungsgemäßen Zusammensetzungen von 5 : 1 bis 1 : 1 und insbesondere bevorzugt von 3 : 1 bis 1 : 1, wobei hierbei Komponente (i) aus der einen oder den mehreren Substanzen der Komponente b) der erfindungsgemäßen Zusammensetzungen und dem einen oder den mehreren Anionen RCOO⁻ der einen oder der mehreren protonierten Zuckeramine aus Komponente a) der erfindungsgemäßen Zusammensetzungen besteht und Komponente (ii) aus dem einen oder den mehreren Zuckeraminen aus Komponente a) der erfindungsgemäßen Zusammensetzungen und dem einen oder den mehreren Kationen der protonierten Zuckeramine aus Komponente a) der erfindungsgemäßen Zusammensetzungen besteht.

In einer weiteren besonders bevorzugten Ausführungsform der Erfindung ist das molare Verhältnis Komponente (i): Komponente (ii) in den erfindungsgemäßen Zusammensetzungen von 1 : 1 bis 1 : 5 und insbesondere bevorzugt von 1 : 1 bis 1 : 3, wobei hierbei Komponente (i) aus der einen oder den mehreren Substanzen der Komponente b) der erfindungsgemäßen Zusammensetzungen und dem einen oder den mehreren Anionen RCOO⁻ der einen oder der mehreren protonierten Zuckeramine aus Komponente a) der erfindungsgemäßen Zusammensetzungen besteht und Komponente (ii) aus dem einen oder den mehreren Zuckeraminen aus Komponente a) der erfindungsgemäßen Zusammensetzungen und dem einen oder den mehreren Kationen der protonierten Zuckeramine aus Komponente a) der erfindungsgemäßen Zusammensetzungen besteht.

Vorzugsweise besitzen die erfindungsgemäßen Zusammensetzungen einen pH-Wert von 7 bis 11 und besonders bevorzugt von 8 bis 10.

Die pH-Werte der erfindungsgemäßen Zusammensetzungen werden direkt in den Zusammensetzungen mit einer Knick Portamess 911 Einstab-Messelektrode nach Kalibrierung mit entsprechenden Standardpuffern gemessen. Die Kalibrierung erfolgt bei pH 4 mit Citronensäure/Natronlauge/Salzsäure-Puffer und bei pH 7 mit Dinatriumhydrogenphosphat, Kaliumdihydrogenphosphat-Puffer.

Vorzugsweise weisen die erfindungsgemäßen Zusammensetzungen eine Viskosität < 500 mPas und besonders bevorzugt < 100 mPas auf.

Die Viskositäten werden mit einem Brookfield-Viskosimeter Model DV II, den Spindeln aus dem Spindelset RV bei 20 Umdrehungen / Minute und 20 °C gemessen. Es werden die Spindeln 1 bis 7 aus dem Spindelset RV verwendet. Unter diesen Messbedingungen wird Spindel 1 für Viskositäten von maximal 500 mPa·s, Spindel 2 für Viskositäten von maximal 1 000 mPa·s, Spindel 3 für Viskositäten von maximal 5 000 mPa·s, Spindel 4 für Viskositäten von maximal 10 000 mPa·s, Spindel 5 für Viskositäten von maximal 20 000 mPa·s, Spindel 6 für Viskositäten von maximal 50 000 mPa·s und Spindel 7 für Viskositäten von maximal 200 000 mPa·s gewählt.

In einer bevorzugten Ausführungsform der Erfindung liegen die erfindungsgemäßen Zusammensetzungen in der Form eines Flüssigwaschmittels vor und besonders bevorzugt in der Form eines hochkonzentrierten Flüssigwaschmittels.

In einer weiteren bevorzugten Ausführungsform der Erfindung liegen die erfindungsgemäßen Zusammensetzungen in der Form eines Duschbades, einer Flüssigseife oder eines Gesichtsreinigers vor.

Anstelle von Zusammensetzungen, die direkt für eine Endanwendung geeignet sind, können die erfindungsgemäßen Zusammensetzungen auch in der Form von Konzentraten vorliegen, die insbesondere zur Herstellung von Zusammensetzungen für bestimmte Endanwendungen verwendet werden können. Die soeben genannten Konzentrate werden im Folgenden als "erfindungsgemäße Konzentrate" bezeichnet. Vorzugsweise ist die Menge der einen oder der mehreren Substanzen der Komponente a) in den erfindungsgemäßen Konzentraten, bezogen auf das Gesamtgewicht der erfindungsgemäßen Konzentrate, von 10 bis 40 Gew.-%. Weiterhin bevorzugt ist die Menge des einen oder der mehreren Substanzen der Komponente b) in den erfindungsgemäßen Konzentraten, bezogen auf das Gesamtgewicht der erfindungsgemäßen Konzentrate, von 5 bis 40 Gew.-%.

Die erfindungsgemäßen Zusammensetzungen, insbesondere die erfindungsgemäßen Konzentrate, eignen sich in vorteilhafter Weise zur Herstellung von Flüssigwaschmitteln und vorzugsweise von hochkonzentrierten Flüssigwaschmitteln. Weiterer Gegenstand der vorliegenden Erfindung ist daher die Verwendung einer erfindungsgemäßen Zusammensetzung, insbesondere eines erfindungsgemäßen Konzentrats, zur Herstellung von Flüssigwaschmitteln und vorzugsweise von hochkonzentrierten Flüssigwaschmitteln.

Die erfindungsgemäßen Zusammensetzungen, insbesondere die erfindungsgemäßen Konzentrate, eignen sich weiterhin in vorteilhafter Weise zur Herstellung von Duschbädern, Flüssigseifen oder Gesichtsreinigern. Weiterer Gegenstand der vorliegenden Erfindung ist daher die Verwendung einer erfindungsgemäßen Zusammensetzung, insbesondere eines erfindungsgemäßen Konzentrats, zur Herstellung von Duschbädern, Flüssigseifen oder Gesichtsreinigern.

Des Weiteren sind die in Komponente a) der erfindungsgemäßen Zusammensetzungen enthaltenen Zuckeramine ausgewählt aus der Gruppe bestehend aus Dimethylglucamin und Hydroxyethyl-methyl-Glucamin und Mischungen davon in vorteilhafter Weise als Neutralisationsmittel für Fettsäuren und vorzugsweise für die in Komponente b) der erfindungsgemäßen Zusammensetzungen enthaltenen Fettsäuren geeignet. Weiterer Gegenstand der vorliegenden Erfindung ist daher auch die Verwendung dieser Zuckeramine als Neutralisationsmittel für Fettsäuren und vorzugsweise für eine oder mehrere Fettsäuren der Formel R-COOH, worin R einen linearen oder verzweigten gesättigten Alkylrest mit 11 bis 21 Kohlenstoffatomen oder einen linearen oder verzweigten einfach oder mehrfach ungesättigten Alkenylrest mit 11 bis 21 Kohlenstoffatomen bedeutet.

Bei der erfindungsgemäßen Verwendung als Neutralisationsmittel ist das Zuckeramin der Formel (I) besonders bevorzugt Dimethylglucamin.

Bei der erfindungsgemäßen Verwendung als Neutralisationsmittel ist die eine oder sind die mehreren Fettsäuren der Formel R-COOH vorzugsweise ausgewählt aus der Gruppe bestehend aus Laurinsäure, Myristinsäure, Palmitinsäure, Stearinsäure, Ölsäure, Linolsäure, C₁₆/C₁₈ Fettsäuren, Behensäure, Erucasäure und Mischungen davon.

Bei der erfindungsgemäßen Verwendung als Neutralisationsmittel ist die eine oder sind die mehreren Fettsäuren der Formel R-COOH besonders bevorzugt ausgewählt aus der Gruppe bestehend aus C₁₆/C₁₈ Fettsäuren und Mischungen davon.

Bei der erfindungsgemäßen Verwendung als Neutralisationsmittel ist die Fettsäure der Formel R-COOH insbesondere bevorzugt Ölsäure.

In einer außerordentlich bevorzugten Ausführungsform der erfindungsgemäßen Verwendung als Neutralisationsmittel ist das Zuckeramin Dimethylglucamin und die Fettsäure der Formel R-COOH Ölsäure.

Bei der erfindungsgemäßen Verwendung als Neutralisationsmittel ist das molare Verhältnis der einen oder der mehreren Fettsäuren der Formel R-COOH zu dem einen oder den mehreren Zuckeraminen ausgewählt aus der Gruppe bestehend aus Dimethylglucamin und Hydroxyethyl-methyl-Glucamin vorzugsweise von 5 : 1 bis 1 : 5 und besonders bevorzugt von 3 : 1 bis 1 : 3.

Die Salze der Formel (la) eignen sich in vorteilhafter Weise als Tenside. Darüber hinaus weisen sie beispielsweise den Vorteil auf, dass sie sich sehr gut in Wasser lösen.

Bei den Salzen handelt sich insbesondere um die Salze der Formel (la) wobei R einen linearen oder verzweigten gesättigten Alkylrest mit 11 bis 21 Kohlenstoffatomen oder einen linearen oder verzweigten einfach oder mehrfach ungesättigten Alkenylrest mit 11 bis 21 Kohlenstoffatomen bedeutet.

Die Salze der Formel (la) sind ausgewählt aus der Gruppe bestehend aus Dimethylglucammonium mit dem Gegenion R-COO⁻, Hydroxyethyl-methyl Glucammonium mit dem Gegenion R-COO⁻ und Mischungen davon.

Besonders bevorzugt sind die Salze der Formel (la) ausgewählt aus der Gruppe bestehend aus Dimethylglucammonium mit dem Gegenion R-COO⁻ und Mischungen davon.

Vorzugsweise ist das Gegenion R-COO⁻ in den Salzen der Formel (Ia) ausgewählt aus der Gruppe bestehend aus den Fettsäuren Laurinsäure, Myristinsäure, Palmitinsäure, Stearinsäure, Ölsäure, Linolsäure, C₁₆/C₁₈ Fettsäuren, Behensäure und Erucasäure, jeweils in deprotonierter Form, und Mischungen davon. Besonders bevorzugt ist das Gegenion R-COO⁻ in den Salzen der Formel (la) ausgewählt aus der Gruppe bestehend aus C₁₆/C₁₈ Fettsäuren, jeweils in deprotonierter Form, und Mischungen davon.

Insbesondere bevorzugt ist das Gegenion R-COO⁻ in den Salzen der Formel (la) Ölsäure in deprotonierter Form.

Außerordentlich bevorzugt handelt es sich bei dem Salz der Formel (la) um Dimethylglucammonium mit deprotonierter Ölsäure als Gegenion R-COO⁻.

Im Folgenden wird die Erfindung anhand von Beispielen näher erläutert, wobei diese jedoch nicht als beschränkend zu verstehen sind. Sofern nicht explizit anders angegeben, sind alle Prozentangaben als Gewichtsprozent (Gew.-%) zu verstehen.

Das in den Beispielen verwendete Dimethylglucamin wurde nach EP 614 881 aus N-Methyl-glucamin durch reduktive Aminierung erhalten.

### Beispiel 1:

5 g Ölsäure wurden in 95 g Wasser vorgelegt (zweiphasig) und mit steigenden molaren Mengen von verschiedenen Neutralisationsmitteln bis zu 100 mol-% Neutralisationsmittel versetzt. Dabei wurde die Viskosität bestimmt (Brookfield, 20°C, 20 Umdrehungen pro Minute (rpm), Spindel 1 oder 2), die Homogenität der entstehenden Lösung beobachtet und der pH-Wert gemessen (siehe Tabelle 1).

Folgende Werte sind in Tabelle 1 aufgeführt bzw. folgende Abkürzungen wurden darin verwendet:
Die obere Zahl in den jeweiligen Zeilen von Tabelle 1 gibt die gemessene Viskosität in mPa·s wieder.

"Z" bedeutet: Die Lösung ist zweiphasig.
"K" bedeutet: Die Lösung ist klar.
"H" bedeutet: Die Lösung ist homogen, aber trüb.
Die untere Zahl in den jeweiligen Zeilen von Tabelle 1 gibt den gemessenen pH-Wert wieder. Hierbei bedeutet "n.b." nicht bestimmt.

**Tabelle 1 verwendete Neutralisationsmittel sowie Viskosität, Homogenität und pH-Wert von hergestellten Zusammensetzungen**

| Neutralisationsmittel [mol-%] | 10 | 20 | 30 | 40 | 50 | 60 | 70 | 80 | 90 | 100 |
|---|---|---|---|---|---|---|---|---|---|---|
| | | | | | | | | | | |
| Monoethanolamin (Vergleich) | 10 | 8 | 8 | 625 | 1060 | 870 | 540 | 28 | 600 | 825 |
| | Z | Z | Z | H | H | H | H | H | H | H |
| | 7,3 | 7,7 | 7,7 | 7,7 | 7,7 | 8,7 | 8,7 | 9,2 | 9,3 | 9,5 |
| Triethanolamin (Vergleich) | 4 | 6 | 104 | 1440 | 1220 | 980 | 910 | 750 | 470 | 420 |
| | Z | Z | Z | H | H | H | H | H | H | H |
| | n.b. | n.b. | n.b. | n.b. | n.b. | n.b. | n.b. | n.b. | n.b. | n.b. |
| Aminomethylpropanol (Vergleich) | 6 | 6 | 9 | 560 | 535 | 183 | 70 | 160 | 2860 | 4200 |
| | Z | Z | Z | H | H | H | H | H | H | K |
| | n.b. | n.b. | n.b. | n.b. | n.b. | n.b. | n.b. | n.b. | n.b. | n.b. |
| Natronlauge (Vergleich) | 6 | 6 | 8 | 13 | 38 | 23 | 13 | 9 | 6 | 6 |
| | Z | Z | Z | Z | H | H | H | Z | Z | K |
| | 8,3 | 8,3 | 8,2 | 8,5 | 8,6 | 9,2 | 9,6 | 9,7 | 9,7 | 11,3 |
| Dimethylglucamin (Erfindung) | 5 | 15 | 25 | 70 | 30 | 20 | 20 | 10 | 10 | 10 |
| | Z | H | H | H | H | H | H | H | H | K |
| | 7,1 | 8,0 | 8,1 | 8,1 | 8,5 | 8,9 | 8,8 | 8,9 | 8,8 | 8,8 |

Aus den Werten der Tabelle 1 ist zu sehen, dass bei Neutralisation von Ölsäure mit Dimethylglucamin schon bei geringen Neutralisationsgraden niedrigviskose, homogene Lösungen resultieren. Homogene Lösungen werden bereits ab einem pH-Wert von 8,0 erhalten.

### Formulierungsbeispiele:

**Formulierungsbeispiel 1: Hochkonzentriertes Flüssigwaschmittel ("heavy duty liquid")**

| Inhaltsstoff | Menge [Gew.-%] |
|---|---|
| Methylestersulfonat | 6 |
| Lineares Alkylbenzolsulfonat | 3 |
| Natrium Laurylethersulfat (2 EO) | 11 |
| Fettsäure (30 Gew.-% C₁₂, 25 Gew.-% C₁₄, 20 Gew.-% C₁₆, 25 Gew.-% C₁₈ ungesättigt) | 3 |
| C₁₂/C₁₅ Alkylethoxylat (8 EO) | 15 |
| Wasser | 51,5 |
| Propylenglykol | 2 |
| Dimethylglucamin | 2 |
| Enzyme | 0,7 |
| Polycarboxylat | 0,6 |
| Natriumcitrat | 5 |
| Borax | 0,2 |
| Natriumhydroxid auf pH 7,7 | |

**Formulierungsbeispiel 2: Hochkonzentriertes Flüssigwaschmittel ("heavy duty liquid")**

| Inhaltsstoff | Menge [Gew.-%] |
|---|---|
| Lineares Alkylbenzolsulfonat | 8 |
| Natrium Laurylethersulfat (2 EO) | 9 |
| Fettsäure (30 Gew.-% C₁₂, 20 Gew.-% C₁₄, 15 Gew.-% C₁₆, 5 Gew.-% C₁₈ gesättigt, 30 Gew.-% C₁₈ ungesättigt) | 5 |
| C₁₂/C₁₄ Alkylethoxylat (7 EO) | 8 |
| Wasser | 48,5 |
| Ethanol | 2 |
| Propylenglykol | 6 |
| Dimethylglucamin | 5 |
| Enzyme | 2 |
| Soil Release Polyester | 0,7 |
| Natriumcitrat | 5 |
| Borax | 0,8 |
| Natriumhydroxid auf pH 8,5 | |

**Formulierungsbeispiel 3: Hochkonzentrieres Flüssigwaschmittel in Monodoseformat ("Monodose Surfactant Capsule)**

| Inhaltsstoff | Menge [Gew.-%] |
|---|---|
| Lineares Alkylbenzolsulfonat | 24 |
| Fettsäure (30 Gew.-% C₁₂, 20 Gew.-% C₁₄, 15 Gew.-% C₁₆, 5 Gew.-% C₁₈ gesättigt, 30 Gew.-% C₁₈ ungesättigt) | 23 |
| C₁₀ Alkylethoxylat (10 EO) | 19 |
| Wasser | 4 |
| Ethanol | 4 |
| Propylenglykol | 10 |
| Dimethylglucamin | 8 |
| Enzyme | 0,7 |
| Complexing agent | 0,5 |
| Methylpropanediol | 6,8 |
| Natriumhydroxid auf pH 8,4 | |

**Formulierungsbeispiel 4: Duschbad / Flüssigseife**

| Inhaltsstoff | Menge [Gew.-%] |
|---|---|
| Laurinsäure | 6 |
| Myristinsäure | 4 |
| Palmitinsäure | 2 |
| Dimethylglucamin | 5 |
| Natriumcocoylglutamat | 2 |
| Cocamide MEA (Cocosfettsäuremonoethanolamid) | 3 |
| Hydroxypropylmethylcellulose | 0,2 |
| Wasser | ad 100 |
| pH mit Kaliumhydroxidlösung auf pH = 8,6 | |

**Formulierungsbeispiel 5: Gesichtsreiniger**

| Inhaltsstoff | Menge [Gew.-%] |
|---|---|
| Laurinsäure | 6 |
| Myristinsäure | 4 |
| Palmitinsäure | 2 |
| Dimethylglucamin | 5 |
| Natriumcocoylglutamat | 2 |
| Oleoyl Methyl Glucamide | 3 |
| Hydroxypropylmethylcellulose | 0,2 |
| Wasser | ad 100 |
| pH mit Kaliumhydroxidlösung auf pH = 8,5 | |

## Patentansprüche

1. Zusammensetzung enthaltend
a) eine oder mehrere Substanzen ausgewählt aus der Gruppe bestehend aus den Zuckeraminen Dimethylglucamin und Hydroxyethyl-methyl Glucamin, den protonierten Zuckeraminen Dimethylglucammonium mit dem Gegenion R-COO⁻ und Hydroxyethyl-methyl Glucammonium mit dem Gegenion R-COO⁻ und Mischungen davon
wobei
R die Bedeutung von R aus den Substanzen der Komponente b) unten besitzt und
b) eine oder mehrere Substanzen ausgewählt aus der Gruppe bestehend aus Fettsäuren der Formel R-COOH, Fettsäuresalzen der Formel R-COO⁻M⁺ und Mischungen davon, worin
R einen linearen oder verzweigten gesättigten Alkylrest mit 11 bis 21 Kohlenstoffatomen oder einen linearen oder verzweigten einfach oder mehrfach ungesättigten Alkenylrest mit 11 bis 21 Kohlenstoffatomen bedeutet und
M⁺ ein Gegenion ist, vorzugsweise ausgewählt aus der Gruppe bestehend aus NH₄⁺, organischen Ammoniumionen [HNR⁵R⁶R⁷]⁺, wobei R⁵, R⁶ und R⁷ unabhängig voneinander Wasserstoff, eine lineare oder verzweigte gesättigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine C₆-C₂₂-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste R⁵, R⁶ und R⁷ nicht Wasserstoff ist, Li⁺, Na⁺, K⁺, ½ Ca⁺⁺, ½ Mg⁺⁺, ½ Zn⁺⁺ oder ⅓ Al⁺⁺⁺ und Mischungen aus diesen Ionen.

2. Zusammensetzung nach Anspruch 1, **dadurch gekennzeichnet, dass** die eine oder die mehreren Substanzen der Komponente a) ausgewählt sind aus der Gruppe bestehend aus Dimethylglucamin, Dimethylglucammonium mit dem Gegenion R-COO⁻ und Mischungen davon.

3. Zusammensetzung nach Anspruch 1 oder 2, **dadurch gekennzeichnet, dass** das Gegenion M⁺ ausgewählt ist aus der Gruppe bestehend aus NH₄⁺, Monoethanolammonium, Diethanolammonium, Triethanolammonium, Methylpropanolammonium, Na⁺, K⁺ und Mischungen aus diesen Ionen und vorzugsweise ausgewählt ist aus der Gruppe bestehend aus Na⁺, K⁺ und Mischungen aus diesen Ionen.

4. Zusammensetzung nach einem oder mehreren der Ansprüche 1 bis 3, **dadurch gekennzeichnet, dass** die eine oder die mehreren Substanzen der Komponente b) ausgewählt sind aus der Gruppe bestehend aus den Fettsäuren (R-COOH) Laurinsäure, Myristinsäure, Palmitinsäure, Stearinsäure, Ölsäure, Linolsäure, C₁₆/C₁₈ Fettsäuren, Behensäure und Erucasäure, den Salzen der genannten Fettsäuren mit M⁺ als Gegenion (R-COO⁻M⁺) und Mischungen davon.

5. Zusammensetzung nach einem oder mehreren der Ansprüche 1 bis 4, **dadurch gekennzeichnet, dass** die eine oder die mehreren Substanzen der Komponente b) ausgewählt sind aus der Gruppe bestehend aus C₁₆/C₁₈ Fettsäuren, den Salzen von C₁₆/C₁₈ Fettsäuren mit M⁺ als Gegenion und Mischungen davon.

6. Zusammensetzung nach einem oder mehreren der Ansprüche 1 bis 5, **dadurch gekennzeichnet, dass** die eine oder die mehreren Substanzen der Komponente b) ausgewählt sind aus der Gruppe bestehend aus Ölsäure, den Salzen von Ölsäure mit M⁺ als Gegenion und Mischungen davon.

7. Zusammensetzung nach einem oder mehreren der Ansprüche 1 bis 6, **dadurch gekennzeichnet, dass** Komponente b) eine oder mehrere Fettsäuren der Formel R-COOH enthält und vorzugsweise keine Fettsäuresalze der Formel R-COO⁻M⁺ enthält.

8. Zusammensetzung nach einem oder mehreren der Ansprüche 1 bis 7, **dadurch gekennzeichnet, dass** sie Wasser enthält.

9. Zusammensetzung nach einem oder mehreren der Ansprüche 1 bis 8, **dadurch gekennzeichnet, dass** sie neben den Substanzen der Komponente a) und den Substanzen der Komponente b) ein oder mehrere weitere anionische Tenside enthält.

10. Zusammensetzung nach Anspruch 9, **dadurch gekennzeichnet, dass** das eine oder die mehreren weiteren anionischen Tenside ausgewählt sind aus der Gruppe bestehend aus Alkyl- oder Alkenylpolyglykolethersulfat, vorzugsweise Alkylpolyglykolethersulfat, Alkyl- oder Alkenylsulfat, vorzugsweise Alkylsulfat, Methylestersulfonat, Alkylbenzolsulfonat, Acylglutamat und Mischungen davon.

11. Zusammensetzung nach einem oder mehreren der Ansprüche 1 bis 10, **dadurch gekennzeichnet, dass** sie ein oder mehrere nichtionische Tenside ausgewählt aus der Gruppe bestehend aus Alkyl- oder Alkenylethoxylaten, vorzugsweise Alkylethoxylaten, Alkylpolyglucosiden, Acyl-N-Methylglucaminen, Fettsäurealkanolamiden und Mischungen davon enthält.

12. Zusammensetzung nach einem oder mehreren der Ansprüche 1 bis 11, **dadurch gekennzeichnet, dass** sie, bezogen auf das Gesamtgewicht der Zusammensetzung, 0,5 bis 30 Gew.-%, vorzugsweise 0,5 bis 20 Gew.-%, besonders bevorzugt 1 bis 15 Gew.-% und insbesondere bevorzugt 1 bis 10 Gew.-% an der einen oder den mehreren Substanzen der Komponente a) enthält.

13. Zusammensetzung nach einem oder mehreren der Ansprüche 1 bis 12, **dadurch gekennzeichnet, dass** sie, bezogen auf das Gesamtgewicht der Zusammensetzung, 0,5 bis 30 Gew.-% und vorzugsweise 1 bis 10 Gew.-% an der einen oder den mehreren Substanzen der Komponente b) enthält.

14. Zusammensetzung nach einem oder mehreren der Ansprüche 1 bis 13, **dadurch gekennzeichnet, dass** das molare Verhältnis Komponente (i) :
Komponente (ii) in der Zusammensetzung von 5 : 1 bis 1 : 5 und vorzugsweise von 3 : 1 bis 1 : 3 ist, wobei hierbei Komponente (i) aus der einen oder den mehreren Substanzen der Komponente b) der Zusammensetzung und dem einen oder den mehreren Anionen R-COO⁻ der einen oder der mehreren protonierten Zuckeramine aus Komponente a) der Zusammensetzung besteht und Komponente (ii) aus dem einen oder den mehreren Zuckeraminen aus Komponente a) der Zusammensetzung und dem einen oder den mehreren Kationen der protonierten Zuckeramine aus Komponente a) der Zusammensetzung besteht.

15. Zusammensetzung nach einem oder mehreren der Ansprüche 1 bis 14, **dadurch gekennzeichnet, dass** sie einen pH-Wert von 7 bis 11 und vorzugsweise von 8 bis 10 besitzt.

16. Zusammensetzung nach einem oder mehreren der Ansprüche 1 bis 15, **dadurch gekennzeichnet, dass** sie eine Viskosität < 500 mPas und vorzugsweise < 100 mPas aufweist.

17. Zusammensetzung nach einem oder mehreren der Ansprüche 1 bis 16, **dadurch gekennzeichnet, dass** sie in der Form eines Flüssigwaschmittels vorliegt.

18. Zusammensetzung nach einem oder mehreren der Ansprüche 1 bis 16, **dadurch gekennzeichnet, dass** sie in der Form eines Duschbades, einer Flüssigseife oder eines Gesichtsreinigers vorliegt.

19. Verwendung einer Zusammensetzung gemäß einem oder mehreren der Ansprüche 1 bis 16, und vorzugsweise einer Zusammensetzung, worin die Menge der einen oder der mehreren Substanzen der Komponente a), bezogen auf das Gesamtgewicht der erfindungsgemäßen Zusammensetzung, bevorzugt von 10 bis 40 Gew.-% ist, und worin die Menge der einen oder der mehreren Substanzen der Komponente b), bezogen auf das Gesamtgewicht der erfindungsgemäßen Zusammensetzung, weiterhin bevorzugt von 5 bis 40 Gew.-% ist, zur Herstellung von Flüssigwaschmitteln.

20. Verwendung einer Zusammensetzung gemäß einem oder mehreren der Ansprüche 1 bis 16, und vorzugsweise einer Zusammensetzung, worin die Menge der einen oder der mehreren Substanzen der Komponente a), bezogen auf das Gesamtgewicht der erfindungsgemäßen Zusammensetzung, bevorzugt von 10 bis 40 Gew.-% ist, und worin die Menge der einen oder der mehreren Substanzen der Komponente b), bezogen auf das Gesamtgewicht der erfindungsgemäßen Zusammensetzung, weiterhin bevorzugt von 5 bis 40 Gew.-% ist, zur Herstellung von Duschbädern, Flüssigseifen oder Gesichtsreinigern.

21. Verwendung einer oder mehrerer Zuckeramine ausgewählt aus der Gruppe bestehend aus Dimethylglucamin, Hydroxyethyl-methyl Glucamin und Mischungen davon als Neutralisationsmittel für Fettsäuren und vorzugsweise für eine oder mehrere Fettsäuren der Formel R-COOH, worin R einen linearen oder verzweigten gesättigten Alkylrest mit 11 bis 21 Kohlenstoffatomen oder einen linearen oder verzweigten einfach oder mehrfach ungesättigten Alkenylrest mit 11 bis 21 Kohlenstoffatomen bedeutet.

22. Verwendung nach Anspruch 21, **dadurch gekennzeichnet, dass** das Zuckeramin Dimethylglucamin ist.

23. Verwendung nach Anspruch 21 oder 22, **dadurch gekennzeichnet, dass** die eine oder die mehreren Fettsäuren der Formel R-COOH ausgewählt sind aus der Gruppe bestehend aus Laurinsäure, Myristinsäure, Palmitinsäure, Stearinsäure, Ölsäure, Linolsäure, C₁₆/C₁₈ Fettsäuren, Behensäure, Erucasäure und Mischungen davon.

24. Verwendung nach einem oder mehreren der Ansprüche 21 bis 23, **dadurch gekennzeichnet, dass** die eine oder die mehreren Fettsäuren der Formel R-COOH ausgewählt sind aus der Gruppe bestehend aus C₁₆/C₁₈ Fettsäuren und Mischungen davon.

25. Verwendung nach einem oder mehreren der Ansprüche 21 bis 24, **dadurch gekennzeichnet, dass** die Fettsäure der Formel R-COOH Ölsäure ist.

26. Verwendung nach einem oder mehreren der Ansprüche 21 bis 25, **dadurch gekennzeichnet, dass** das molare Verhältnis der einen oder der mehreren Fettsäuren der Formel R-COOH zu dem einen oder den mehreren Zuckeraminen von 5 : 1 bis 1 : 5 und vorzugsweise von 3 : 1 bis 1 : 3 ist.

## Claims

1. Composition comprising
a) one or more substances selected from the group consisting of the sugar amines dimethylglucamine and hydroxyethylmethylglucamine, the protonated sugar amines dimethylglucammonium with the counterion R-COO⁻ and hydroxyethylmethylglucammonium with the counterion R-COO⁻ and mixtures thereof
where
R has the meaning of R from the substances of component b) below
and
b) one or more substances selected from the group consisting of fatty acids of the formula R-COOH, fatty acid salts of the formula R-COO⁻M⁺ and mixtures thereof, in which
R is a linear or branched saturated alkyl radical having 11 to 21 carbon atoms or a linear or branched mono- or polyunsaturated alkenyl radical having 11 to 21 carbon atoms and
M⁺ is a counterion, preferably selected from the group consisting of NH₄⁺, organic ammonium ions [HNR⁵R⁶R⁷]⁺, where R⁵, R⁶ and R⁷, independently of one another, can be a linear or branched saturated alkyl group having 1 to 22 carbon atoms, a linear or branched, mono- or polyunsaturated alkenyl group having 2 to 22 carbon atoms, a C₆-C₂₂-alkylamidopropyl group, a linear monohydroxyalkyl group having 2 to 10 carbon atoms or a linear or branched dihydroxyalkyl group having 3 to 10 carbon atoms, and where at least one of the radicals R⁵, R⁶ and R⁷ is not hydrogen, Li⁺, Na⁺, K⁺, ½ Ca⁺⁺, ½ Mg⁺⁺, ½ Zn⁺⁺ or ⅓ Al⁺⁺⁺ and mixtures of these ions.

2. Composition according to Claim 1, **characterized in that** the one or more substances of component a) are selected from the group consisting of dimethylglucamine, dimethylglucammonium with the counterion R-COO⁻ and mixtures thereof.

3. Composition according to Claim 1 or 2, **characterized in that** the counterion M⁺ is selected from the group consisting of NH₄⁺, monoethanolammonium, diethanolammonium, triethanolammonium, methylpropanolammonium, Na⁺, K⁺ and mixtures of these ions and is preferably selected from the group consisting of Na⁺, K⁺ and mixtures of these ions.

4. Composition according to one or more of Claims 1 to 3, **characterized in that** the one or more substances of component b) are selected from the group consisting of the fatty acids (R-COOH) lauric acid, myristic acid, palmitic acid, stearic acid, oleic acid, linoleic acid, C₁₆/C₁₈ fatty acids, behenic acid and erucic acid, the salts of the specified fatty acids with M⁺ as counterion (R-COO⁻M⁺) and mixtures thereof.

5. Composition according to one or more of Claims 1 to 4, **characterized in that** the one or more substances of component b) are selected from the group consisting of C₁₆/C₁₈ fatty acids, the salts of C₁₆/C₁₈ fatty acids with M⁺ as counterion and mixtures thereof.

6. Composition according to one or more of Claims 1 to 5, **characterized in that** the one or more substances of component b) are selected from the group consisting of oleic acid, the salts of oleic acid with M⁺ as counterion and mixtures thereof.

7. Composition according to one or more of Claims 1 to 6, **characterized in that** component b) comprises one or more fatty acids of the formula R-COOH and preferably comprises no fatty acid salts of the formula R-COO⁻M⁺.

8. Composition according to one or more of Claims 1 to 7, **characterized in that** it comprises water.

9. Composition according to one or more of Claims 1 to 8, **characterized in that** it comprises one or more further anionic surfactants besides the substances of component a) and the substances of component b).

10. Composition according to Claim 9, **characterized in that** the one or more further anionic surfactants are selected from the group consisting of alkyl- or alkenylpolyglycol ether sulfate, preferably alkylpolyglycol ether sulfate, alkyl or alkenyl sulfate, preferably alkyl sulfate, methyl ester sulfonate, alkylbenzenesulfonate, acylglutamate and mixtures thereof.

11. Composition according to one or more of Claims 1 to 10, **characterized in that** it comprises one or more nonionic surfactants selected from the group consisting of alkyl or alkenyl ethoxylates, preferably alkyl ethoxylates, alkylpolyglucosides, acyl-N-methylglucamines, fatty acid alkanolamides and mixtures thereof.

12. Composition according to one or more of Claims 1 to 11, **characterized in that** it comprises, based on the total weight of the composition, 0.5 to 30% by weight, preferably 0.5 to 20% by weight, particularly preferably 1 to 15% by weight and especially preferably 1 to 10% by weight, of the one or more substances of component a).

13. Composition according to one or more of Claims 1 to 12, **characterized in that** it comprises, based on the total weight of the composition, 0.5 to 30% by weight and preferably 1 to 10% by weight of the one or more substances of component b).

14. Composition according to one or more of Claims 1 to 13, **characterized in that** the molar ratio of component (i) : component (ii) in the composition is from 5 : 1 to 1 : 5 and preferably from 3 : 1 to 1 : 3, where herein component (i) consists of the one or more substances of component b) of the composition and the one or more anions R-COO⁻ of the one or more protonated sugar amines from component a) of the composition, and component (ii) consists of the one or more sugar amines from component a) of the composition and the one or more cations of the protonated sugar amines from component a) of the composition.

15. Composition according to one or more of Claims 1 to 14, **characterized in that** it has a pH of from 7 to 11 and preferably from 8 to 10.

16. Composition according to one or more of Claims 1 to 15, **characterized in that** it has a viscosity < 500 mPas and preferably < 100 mPas.

17. Composition according to one or more of Claims 1 to 16, **characterized in that** it is in the form of a liquid detergent.

18. Composition according to one or more of Claims 1 to 16, **characterized in that** it is in the form of a shower bath, a liquid soap or a face cleanser.

19. Use of a composition according to one or more of Claims 1 to 16, and preferably a composition in which the amount of the one or more substances of component a), based on the total weight of the composition according to the invention, is preferably from 10 to 40% by weight, and in which the amount of the one or more substances of the component b), based on the total weight of the composition according to the invention, is furthermore preferably from 5 to 40% by weight, for producing liquid detergents.

20. Use of a composition according to one or more of Claims 1 to 16, and preferably of a composition in which the amount of the one or more substances of component a), based on the total weight of the composition according to the invention, is preferably from 10 to 40% by weight, and in which the amount of the one or more substances of component b), based on the total weight of the composition according to the invention, is furthermore preferably from 5 to 40% by weight, for producing shower baths, liquid soaps or face cleansers.

21. Use of one or more sugar amines selected from the group consisting of dimethylglucamine, hydroxyethylmethylglucamine and mixtures thereof as neutralizing agents for fatty acids and preferably for one or more fatty acids of the formula R-COOH in which R is a linear or branched saturated alkyl radical having 11 to 21 carbon atoms or a linear or branched mono- or polyunsaturated alkenyl radical having 11 to 21 carbon atoms.

22. Use according to Claim 21, **characterized in that** the sugar amine is dimethylglucamine.

23. Use according to Claim 21 or 22, **characterized in that** the one or more fatty acids of the formula R-COOH are selected from the group consisting of lauric acid, myristic acid, palmitic acid, stearic acid, oleic acid, linoleic acid, C₁₆/C₁₈ fatty acids, behenic acid, erucic acid and mixtures thereof.

24. Use according to one or more of Claims 21 to 23, **characterized in that** the one or more fatty acids of the formula R-COOH are selected from the group consisting of C₁₆/C₁₈ fatty acids and mixtures thereof.

25. Use according to one or more of Claims 21 to 24, **characterized in that** the fatty acid of the formula R-COOH is oleic acid.

26. Use according to one or more of Claims 21 to 25, **characterized in that** the molar ratio of the one or more fatty acids of the formula R-COOH to the one or more sugar amines is from 5 : 1 to 1 : 5 and preferably from 3 : 1 to 1 : 3.

## Revendications

1. Composition contenant
a) une ou plusieurs substances choisies dans le groupe constitué par les amines de sucre diméthylglucamine et hydroxyéthylméthylglucamine, les amines de sucre protonées diméthylglucammonium présentant le contre-ion R-COO⁻ et hydroxyéthylméthylglucammonium présentant le contre-ion R-COO⁻ et leurs mélanges, où
R possède la signification de R des substances du composant b) ci-dessous et
b) une ou plusieurs substances choisies dans le groupe constitué par les acides gras de formule R-COOH, les sels d'acide gras de formule R-COO⁻M⁺ et leurs mélanges, où
R signifie un radical alkyle saturé, linéaire ou ramifié, comprenant 11 à 21 atomes de carbone ou un radical alcényle monoinsaturé ou polyinsaturé, linéaire ou ramifié comprenant 11 à 21 atomes de carbone et
M⁺ représente un contre-ion, de préférence choisi dans le groupe constitué par NH₄⁺ ; les ions organiques d'ammonium [HNR⁵R⁶R⁷]⁺, R⁵, R⁶ et R⁷ pouvant représenter, indépendamment les uns des autres, hydrogène, un groupe alkyle saturé linéaire ou ramifié, comprenant 1 à 22 atomes de carbone, un groupe alcényle monoinsaturé ou polyinsaturé, linéaire ou ramifié, comprenant 2 à 22 atomes de carbone, un groupe C₆-C₂₂-alkylamidopropyle, un groupe monohydroxyalkyle linéaire comprenant 2 à 10 atomes de carbone ou un groupe dihydroxyalkyle linéaire ou ramifié comprenant 3 à 10 atomes de carbone et au moins un des radicaux R⁵, R⁶ et R⁷ ne représentant pas hydrogène ; Li⁺, Na⁺, K⁺, 1/2 Ca⁺⁺, 1/2 Mg⁺⁺, 1/2 Zn⁺⁺ ou 1/3 Al⁺⁺⁺ et les mélanges de ces ions.

2. Composition selon la revendication 1, **caractérisée en ce que** ladite une ou lesdites plusieurs substances du composant a) sont choisies dans le groupe constitué par la diméthylglucamine, le diméthylglucammonium présentant le contre-ion R-COO⁻ et leurs mélanges.

3. Composition selon la revendication 1 ou 2, **caractérisée en ce que** le contre-ion M⁺ est choisi dans le groupe constitué par NH₄⁺, le monoéthanolammonium, le diéthanolammonium, le triéthanolammonium, le méthylpropanolammonium, Na⁺, K⁺ et les mélanges de ces ions et de préférence choisi dans le groupe constitué par Na⁺, K⁺ et les mélanges de ces ions.

4. Composition selon l'une ou plusieurs des revendications 1 à 3, **caractérisée en ce que** ladite une ou lesdites plusieurs substances du composant b) sont choisies dans le groupe constitué par les acides gras (R-COOH) acide laurique, acide myristique, acide palmitique, acide stéarique, acide oléique, acide linoléique, acides gras en C₁₆/C₁₈, acide béhénique et acide érucasique, les sels des acides mentionnés présentant M⁺ comme contre-ion (R-COO⁻M⁺) et leurs mélanges.

5. Composition selon l'une ou plusieurs des revendications 1 à 4, **caractérisée en ce que** ladite une ou lesdites plusieurs substances du composant b) sont choisies dans le groupe constitué par les acides gras en C₁₆/C₁₈, les sels des acides gras en C₁₆/C₁₈ présentant M⁺ comme contre-ion et leurs mélanges.

6. Composition selon l'une ou plusieurs des revendications 1 à 5, **caractérisée en ce que** ladite une ou lesdites plusieurs substances du composant b) sont choisies dans le groupe constitué par l'acide oléique, les sels de l'acide oléique présentant M⁺ comme contre-ion et leurs mélanges.

7. Composition selon l'une ou plusieurs des revendications 1 à 6, **caractérisée en ce que** le composant b) contient un ou plusieurs acides gras de formule R-COOH et ne contient de préférence pas de sels d'acide gras de formule R-COO⁻M⁺.

8. Composition selon l'une ou plusieurs des revendications 1 à 7, **caractérisée en ce qu'**elle contient de l'eau.

9. Composition selon l'une ou plusieurs des revendications 1 à 8, **caractérisée en ce qu'**elle contient, outre les substances du composant a) et les substances du composant b), un ou plusieurs autres tensioactifs anioniques.

10. Composition selon la revendication 9, **caractérisée en ce que** ledit un ou lesdits plusieurs autres tensioactifs anioniques sont choisis dans le groupe constitué par un alkylpolyglycoléthersulfate ou un alcénylpolyglycoléthersulfate, de préférence un alkylpolyglycoléthersulfate, un alkylsulfate ou un alcénylsulfate, de préférence un alkylsulfate, un méthylestersulfonate, un alkylbenzènesulfonate, un acylglutamate et leurs mélanges.

11. Composition selon l'une ou plusieurs des revendications 1 à 10, **caractérisée en ce qu'**elle contient un ou plusieurs tensioactifs non ioniques choisis dans le groupe constitué par les éthoxylates d'alkyle ou d'alcynyle, de préférence les éthoxylates d'alkyle, les alkylpolyglucosides, les acyl-N-méthylglucamines, les alcanolamides d'acide gras et leurs mélanges.

12. Composition selon l'une ou plusieurs des revendications 1 à 11, **caractérisée en ce qu'**elle contient, par rapport au poids total de la composition, 0,5 à 30% en poids, de préférence 0,5 à 20% en poids, de manière particulièrement préférée 1 à 15% en poids et en particulier de préférence 1 à 10% en poids de ladite une ou desdites plusieurs substances du composant a).

13. Composition selon l'une ou plusieurs des revendications 1 à 12, **caractérisée en ce qu'**elle contient, par rapport au poids total de la composition, 0,5 à 30% en poids et de préférence 1 à 10% en poids de ladite une ou desdites plusieurs substances du composant b) .

14. Composition selon l'une ou plusieurs des revendications 1 à 13, **caractérisée en ce que** le rapport molaire composant (i):composant (ii) dans la composition est de 5:1 à 1:5 et de préférence de 3:1 à 1:3, le composant (i) étant constitué par ladite une ou lesdites plusieurs substances du composant b) de la composition et par ledit un ou lesdits plusieurs anions R-COO⁻ de ladite une ou desdites plusieurs amines de sucre protonées du composant a) de la composition et le composant (ii) étant constitué par ladite une ou lesdites plusieurs amines de sucre du composant a) de la composition et par ledit un ou lesdits plusieurs cations des amines de sucre protonées du composant a) de la composition.

15. Composition selon l'une ou plusieurs des revendications 1 à 14, **caractérisée en ce qu'**elle présente une valeur de pH de 7 à 11 et de préférence de 8 à 10.

16. Composition selon l'une ou plusieurs des revendications 1 à 15, **caractérisée en ce qu'**elle présente une viscosité < 500 mPa.s et de préférence < 100 mPa.s.

17. Composition selon l'une ou plusieurs des revendications 1 à 16, **caractérisée en ce qu'**elle se trouve sous forme d'un agent de lavage liquide.

18. Composition selon l'une ou plusieurs des revendications 1 à 16, **caractérisée en ce qu'**elle se trouve sous forme d'un bain-douche, d'un savon liquide ou d'un agent de nettoyage pour visage.

19. Utilisation d'une composition selon l'une ou plusieurs des revendications 1 à 16 et de préférence d'une composition dans laquelle la quantité de ladite une ou desdites plusieurs substances du composant a), par rapport au poids total de la composition selon l'invention, est de préférence de 10 à 40% en poids et dans laquelle la quantité de ladite une ou desdites plusieurs substances du composant b), par rapport au poids total de la composition selon l'invention, est en outre de préférence de 5 à 40% en poids, pour la préparation d'agents de lavage liquides.

20. Utilisation d'une composition selon l'une ou plusieurs des revendications 1 à 16 et de préférence d'une composition dans laquelle la quantité de ladite une ou desdites plusieurs substances du composant a), par rapport au poids total de la composition selon l'invention, est de préférence de 10 à 40% en poids et dans laquelle la quantité de ladite une ou desdites plusieurs substances du composant b), par rapport au poids total de la composition selon l'invention, est en outre de préférence de 5 à 40% en poids, pour la préparation de bains-douches, de savons liquides ou d'agents de nettoyage pour visage.

21. Utilisation d'une ou de plusieurs amines de sucre choisies dans le groupe constitué par la diméthylglucamine, l'hydroxyéthylméthylglucamine et les mélanges de ceux-ci comme agent de neutralisation pour les acides gras et de préférence pour un ou plusieurs acides gras de formule R-COOH, dans laquelle R signifie un radical alkyle saturé, linéaire ou ramifié, comprenant 11 à 21 atomes de carbone ou un radical alcényle monoinsaturé ou polyinsaturé, linéaire ou ramifié, comprenant 11 à 21 atomes de carbone.

22. Utilisation selon la revendication 21, **caractérisée en ce que** l'amine de sucre est la diméthylglucamine.

23. Utilisation selon la revendication 21 ou 22, **caractérisée en ce que** ledit un ou lesdits plusieurs acides gras de formule R-COOH est/sont choisi(s) dans le groupe constitué par l'acide laurique, l'acide myristique, l'acide palmitique, l'acide stéarique, l'acide oléique, l'acide linoléique, les acides gras en C₁₆/C₁₈, l'acide béhénique, l'acide érucasique et leurs mélanges.

24. Utilisation selon l'une ou plusieurs des revendications 21 à 23, **caractérisée en ce que** ledit un ou lesdits plusieurs acides gras de formule R-COOH est/sont choisi(s) dans le groupe constitué par les acides gras en C₁₆/C₁₈ et leurs mélanges.

25. Utilisation selon l'une ou plusieurs des revendications 21 à 24, **caractérisée en ce que** l'acide gras de formule R-COOH est l'acide oléique.

26. Utilisation selon l'une ou plusieurs des revendications 21 à 25, **caractérisée en ce que** le rapport molaire dudit un ou desdits plusieurs acides gras de formule R-COOH à ladite une ou lesdites plusieurs amines de sucre est de 5:1 à 1:5 et de préférence de 3:1 à 1:3.
